# FASCICULE DE BREVET EUROPEEN

(11) **EP 1 587 556 B1**
(45) Date de publication et mention de la délivrance du brevet: **15.10.2008**
(21) Numéro de dépôt: 04705429.1
(22) Date de dépôt: 27.01.2004
(51) Int. Cl.: A61L 27/12, A61L 27/50, A61L 27/58, A61L 27/46, A61F 2/00

(54) **IMPLANTS INJECTABLES A BASE DE CERAMIQUE POUR LE COMBLEMENT DE TISSUS MOUS**
INJIZIERBARE IMPLANTATE AUF KERAMIKBASIS ZUR FÜLLUNG VON WEICHGEWEBE
CERAMIC-BASED INJECTABLE IMPLANTS WHICH ARE USED TO FILL WRINKLES, CUTANEOUS DEPRESSIONS AND SCARS, AND PREPARATION METHOD THEREOF

(30) Priorité: 27.01.2003 FR 0300853
(43) Date de publication de la demande: 26.10.2005
(73) Titulaire: ABR Invent, 30130 Pujaut (FR)
(72) Inventeur: ASIUS, Jérôme, 34670 Baillargues (FR); ASIUS, Bénédicte, 34670 Baillargues (FR)
(74) Mandataire: Pöpping, Barbara
(86) Numéro de dépôt international: PCT/FR2004/000180
(87) Numéro de publication internationale: WO 2004/069090

(56) Documents cités:
- EP-A- 0 196 143
- EP-A- 1 080 698
- WO-A-01/12247
- WO-A-91/17777
- WO-A-93/15721
- WO-A-94/21299
- WO-A-99/02107
- US-A- 5 997 574

## Description

L'invention concerne des implants injectables par voie sous-cutanée ou intradermique dans le tissu fibreux, destinés à être utilisés chez l'homme ou l'animal en chirurgie réparatrice ou plastique ou en dermatologie esthétique pour le comblement de rides, ridules, dépressions cutanées, et cicatrices, y compris le comblement de défects cutanés secondaires à la prise d'un traitement pouvant entraîner une lipodystrophie se caractérisant le plus souvent par une lipoatrophie faciale.

Jusqu'à ce jour, un certain nombre de produits ont été utilisés. Chaque produit présente des avantages et des inconvénients.
• Les huiles de silicone interdites en injections étaient faciles à utiliser. Cependant, il a été constaté, après injection, la migration de gouttelettes de silicone dans les tissus situés en dessous du point d'injection, par simple gravité. La silicone mal utilisée ou utilisée en grande quantité a été la cause de siliconome, et même de réactions allergiques tardives. La silicone n'est pas biodégradable.
• La pâte de Téflon^{®} est une suspension de microparticules de polytetrafluoréthylène (diamètre de 10 à 100 µm,) dans de la glycérine. Ce produit, dans de nombreux cas, a provoqué des infections séreuses sévères et chroniques et a du être retiré au bout de quelques mois des tissus dermiques et sous dermiques pour la plupart des patients. Il a été également prouvé que des petites microparticules de polytetrafluoréthylène ont été retrouvées dans le foie.
• Les suspensions de collagène ont été très largement utilisées dans les dix dernières années. Très longtemps, le collagène est resté leader dans ces indications car il était quasiment le seul produit utilisé et bénéficiant d'une autorisation de mise sur le marché pour le traitement du vieillissement cutané. Il a été noté quelques cas de réactions allergiques chez environ 3% des patients. La résorption du collagène se situe en moyenne chez la plupart des patients entre 3 et 5 mois, ce qui nécessite plusieurs injections par an afin d'avoir une certaine efficacité. Il est enfin à noter que le collagène est d'origine bovine.
• Les prélèvements biologiques du patient lui-même. L'idée était certes intéressante, mais l'expérience clinique a montré l'échec de la réimplantation des cellules graisseuses qui sont absorbées et disparaissent en quelques semaines. Un autre système consistait à ajouter du plasma du patient dans une gélatine de collagène d'origines bovine et porcine. Les résultats sont encore plus décevants et le produit est d'origine animale.
• L'acide hyaluronique utilisé dans la plupart des formes pharmaceutiques ou dans la plupart des dispositifs médicaux se présente sous la forme d'un gel de hyaluronate de sodium. Il est très utilisé grâce à sa facilité d'injection et sa sécurité d'emploi, et il présente une bonne alternative de par sa biocompatibilité et son absence de toxicité. Ces gels de hyaluronate de sodium sont par ailleurs largement utilisés en chirurgie oculaire. Cependant, leur biorésorbabilité rapide (variant typiquement entre 4 et 6 mois) peut décevoir certains utilisateurs dans le domaine du comblement des rides ou dépressions cutanées, car les injections doivent être répétées à intervalles proches et réguliers.
• Les bioplastiques sont des microparticules de silicone polymérisées (diamètre 70 à 140 µ) dispersées dans de la polyvinylpyrrolidone. On a pu noter des réactions de rejet.
• Les microsphères de polyméthylméthacrylate (PMMA) de diamètre 20 à 40 µ en suspension soit dans une solution de gélatine, soit dans une solution de collagène, soit dans une solution d'acide hyaluronique. Le PMMA n'est pas biodégradable, bien que largement utilisé dans le domaine de l'ophtalmologie sous forme d'implant intra-oculaire. Dans le domaine de la dermato-esthétique, on ne dispose pas d'un recul suffisant pour savoir ce que cet implant donnes après cinq ou six ans d'implantation intradermique. Par ailleurs, lorsque le vecteur est une solution de collagène (d'origine bovine), on a pu épertorier 3% de cas d'allergie.
• Les microparticules d'acide poly-lactique (PLA) de forme polymorphe et de diamètre de 40 à 63 µm en suspension dans de la carmellose sodique. Le produit commercialisé sous le nom de NEWFILL^{®} représente un progrès intéressant dans la mesure où il permet une efficacité de traitement dans une durée assez longue en limitant les séances d'injection. Le polymère utilisé est un L PLA 100 (forme cristalline du PLA 100 % lévogyre) impliquant une cinétique de résorption extrêmement lente (au delà de 5 ans). On peut cependant redouter la persistance de cristaux de PLA dans les tissus pouvant, dans certains cas, entraîner à long terme des réactions inflammatoires chroniques lors d'injections itératives. L'utilisation de CMC (dérivé cellulosique) peut d'une part être à l'origine de réactions allergiques, et d'autre part l'organisme ne possède pas de système enzymatique capables de dégrader la cellulose. De plus, une reconstitution extemporanée, une agitation énergique pour homogénéisation avant son utilisation, la mauvaise seringabilité du produit, limitent son utilisation et rebutent un grand nombre d'utilisateurs. On commence à répertorier des cas de granulomes à deux ans ainsi que des nodules kystiques nécessitant dans la plupart des cas une exérèse.

Le but de la présente invention est de remédier aux inconvénients des produits connus existants sur le marché, et en particulier l'utilisation de produits d'origine animale et plus particulièrement bovine, l'obligation d'injection régulières (tous les quelques mois), la manifestation de réactions allergiques, et la difficulté d'une injection simple à opérer.

Pour cela, l'invention fait appel à un composé thixotrope biodégradable ayant des propriétés pseudoplastiques utilisable dans un implant injectable selon l'invention qui permet de stabiliser la suspension et de faciliter notablement l'injection de tout matériau, biodégradable ou non, facilitant la fabrication dudit implant et la seringuabilité dudit implant au travers d'aiguilles fines, typiquement de gauge 25 à 30, préférables pour une utilisation dermatologique et/ou esthétique.

Des agents thixotropes et pseudoplastiques tels que l'hydroxyéthylcellulose, la carboxyméthylcellulose ou la gomme de xanthane, ont déjà été utilisés dans la fabrications d'implants mammaires (US-5,997,574). Il a en outre été suggéré de les utiliser comme matrice pseudoplastique pour la fabrication de produits de comblement des rides (WO 94/21299). Ces produits de comblement comprennent des particules d'un matériau d'implantation tel que le dextranomer, qui favorise le développement de tissu conjonctif tel que le collagène à la surface des particules, sans provoquer d'inflammation.

L' invention fait également appel à un composé de céramique résorbable choisi pour son innocuité et déjà utilisé largement dans le domaine médical, plus particulièrement dans le domaine des implants de tissus osseux (voir notamment la demande EP-0 196 143).

On connaît déjà la famille de brevets et/ou demandes de brevet dont le brevet EP-B1-0.627.899, qui décrit une composition d'implant injectable comprenant une matrice céramique biocompatible présente dans un support fluide pharmaceutiquement acceptable choisi dans le groupe consistant en un milieu aqueux tamponné, des polymères organiques biocompatibles qui se dissiperont à partir d'un site d'injection dans un tissu, et leurs mélanges, dans laquelle la matrice céramique comprend des particules ayant une distribution des dimensions comprise dans l'intervalle de 50 µm à 250 µm. L'implant dudit brevet vise à combler les cavités fibreuses, principalement à proximité de tissu osseux ou de tissu dur. S'il est mentionné que l'implant décrit peut être injecté dans des tissus mous, avec des aiguilles de gauge supérieure à 20, de préférence supérieure à 22, il est par contre (et contradictoirement) aussi indiqué que de préférence, cette injection qui doit aider la croissance de tissus doit se faire avec une aiguille de gauge de 20 ou moins, et auprès d'os ou de cartilage dans le but d'une réparation nasale ou d'une réparation de sphincter. D'autre part, il est bien spécifié que la taille des céramiques, de 50 µm à 250 µm, de préférence de 100 µm à 200 µm, doit permettre l'injection par des aiguilles fines. Au dessous d'une taille de 50 µm, il est indiqué que les particules de céramique auront l'inconvénient d'être soumises à un phagocytage trop important. Au dessus de 200 µm, il est indiqué que les particules seront trop difficilement injectables. Dans l'exemple de réalisation, les implants injectés, et analysés, comprennent de l'hydroxyapatite (HA) mélangée à du collagène. Ils présentent tous un début de calcification. Aucun exemple ne comprend de la céramique seule. En effet il est bien précisé dans le préambule du brevet que le collagène agit pour les comblements sous la surface de la peau, tandis que les particules de céramique sont destinées aux réparations à proximité des os et des cartilages. Or la présence de collagène n'est pas souhaité, ainsi qu'il a été explicité plus haut.

On connaît aussi les documents de HUBBARD WILLIAM G tel que WO-A-93/15.721 et de BIOFORM INC tels que WO-A-01/12.247 et EP-A-1.080.698. Ils décrivent des matériaux biocompatibles et permanents, c'est-à-dire non résorbables, comprenant une matrice de particules céramique, pour l'augmentation de volume de partie molle (ou « soft tissue » en anglais). Lesdites particules sont substantiellement sphériques et ont une taille contrôlée, généralement de 35 à 150µm, mais il est aussi possible que cette taille soit de moins de 35µm, de préférence de 10 à 30µm. Dans WO-A-93/15.721 et WO-A-01/12.247, un matériau céramique préféré est l'hydroxyapatite ou HAP, mais du phosphate tri calcium peut aussi être utilisé. Le véhicule de ces particules est un matériau lubrifiant, biocompatible et résorbable qui comprend un polysaccharide. Dans WO-A-93/15.721, parmi les polysaccharides possibles sont cités le carboxy méthyl cellulose CMC) de sodium et la glycérine, leur combinaison étant particulièrement préférée. Dans WO-A-01/12.247, parmi les polysaccharides possibles sont cités l'acide hyaluronique, mais surtout le carboxy méthyl cellulose (CMC) de sodium et la glycérine, leur combinaison étant particulièrement préférée. La différence essentielle de ces implants avec ceux de la présente invention réside dans le caractère permanent des implants selon cet art antérieur.

Les implants injectables selon l'invention pallient les inconvénients de l'art antérieur. Ils permettent en particulier de combler les rides, ridules, citatrices et/ou dépressions cutanées par un produit simple et efficace, à biorésorbabilité pratiquement totale.

L'invention concerne en premier lieu un implant injectable par voie sous-cutanée ou intradermique dans le tissu fibreux, comprenant des microparticules d'au moins un composé de céramique biocompatible en suspension dans au moins un fluide vecteur, ledit implant étant caractérisé en ce que lesdites microparticules sont biodégradables et ont une taille de 10 à 80 µm, de préférence de 15 à 50 µm, ledit composé de céramique comprenant au moins un élément choisi dans le groupe formé par le phosphate tricalcique (βTCP) et les produits biphasés (BPC) qui comprennent de l'HAP et du βTCP en proportion variable, de préférence ledit élément étant du βTCP, et en ce que ledit fluide vecteur comprend au moins un composé à base d'acide hyaluronique et au moins un composé thixotrope biodégradable ayant des propriétés pseudoplastiques.

Le composé thixotrope biodégradable ayant des propriétés pseudoplastiques utilisé dans l'implant injectable selon l'invention permet de stabiliser la suspension et de faciliter notablement l'injection de tout matériau, biodégradable ou non, facilitant la fabrication dudit implant et la seringuabilité dudit implant au travers d'aiguilles fines, typiquement de gauge 25 à 30, préférables pour une utilisation dermatologique et/ou esthétique.

Par implant, on entend selon l'invention aussi bien une composition destinée à être implantée qu'une composition qui a été implantée dans le corps humain ou animal. Par fluide vecteur, on entend selon l'invention un composé qui véhicule le composé de céramique, et qui est sous forme fluide. Le terme fluide comprend ici aussi un gel par exemple viscoélastique. On entend par gel selon l'invention une structure physique tridimensionnelle ayant des propriétés viscosifiantes, rhéologiques et thixotropiques. Un tel gel comprend donc la présence d'au moins un composé thixotrope ayant des propriétés pseudoplastiques. Par tissu fibreux, on entend selon l'invention un espace sous-cutané de nature essentiellement fibreuse, et apte à être rempli par des fibres. Par sous-cutanée on entend selon l'invention hypodermique, donc sous le derme. Par intradermique on entend selon l'invention dans l'épaisseur du derme. Par en suspension, on entend selon l'invention l'état d'une poudre dispersée dans un fluide et non soluble dans ledit fluide.

Par biodégradation ou dégradation, on entend selon l'invention altération dans un environnement biologique d'un matériau résultant d'une activité cellulaire, enzymatique, bactérienne, virale. La biodégradation correspond à la perte des propriétés physiques. Par biorésorption ou résorption, on entend selon l'invention une biodégradation qui aboutit à la disparition complète du matériau, les produits de dégradation étant généralement éliminés par voie rénale ou métabolisés.

L'implant selon l'invention comprend au moins un composé thixotrope biodégradable ayant des propriétés pseudoplastiques, de préférence d'au moins un composé thixotrope biorésorbable ayant des propriétés pseudoplastiques, de façon encore plus préférée d'au moins un composé thixotrope ayant des propriétés pseudoplastiques à base de gomme de xanthane. Par exemple un tel composé est du xanthural 180® de la société CPKELCO Inc.

Dans le mode de réalisation préféré de l'implant selon l'invention, les microparticules de composé de céramique sont généralement résorbables (ou pratiquement résorbables), une fois l'implantation effectuée dans le tissu fibreux, dans un délai de 2 à 36 mois, de préférence de 3 à 24 mois, et de façon encore plus préférée de 4 à 18 mois. On parle alors de céramique résorbable. Bien entendu le fluide vecteur est choisi tel qu'il soit lui-même biodégradable et de préférence biorésorbable, et aussi compatible avec les propriétés de résorbabilité du composé de céramique. Dans tous les cas on veillera généralement, selon l'invention, à éviter les vecteurs d'origine animale tel que le collagène bovin.

La taille des microparticules est une taille moyenne, mesurée par tamisage (typiquement par une méthode par aspiration et vibration à l'aide de tamis normalisé selon l'AFNOR) ou par granulométrie laser. Les pourcentages d'erreurs sont approximativement et généralement de l'ordre d'environ 10% par tamisage et d'environ 2% par granulométrie laser. De préférence, les microparticules ont généralement une taille comprise entre 10 et 80 µm, de façon préférée de 10 à 50 µm. De façon encore plus préférée, les microparticules ont une taille de 10 à 45 µm, de préférence de 15 à 40 µm.

Toute forme de microparticules entre dans le cadre de la présente invention. Selon une variante, les microparticules peuvent être sensiblement des microsphères.

Selon l'invention, on entend par microparticules des microparticules enrobées ou non par un excipient biocompatible et connu de l'homme du métier.

Ainsi, selon l'invention, les microparticules de céramique résorbables sont une matière ni organique ni métallique ayant généralement subi un traitement de cuisson en température et en pression (frittage). La structure générale des céramiques est biphasique : phase vitreuse (matrice), et phase cristalline (aiguilles cristallisées). Les céramiques traditionnelles sont généralement des produits de terre cuite, porcelaines, faiences, verres etc. Les céramiques nouvelles, elles, plus intéressantes dans le cadre de l'invention, ont en commun des propriétés intéressantes telles que haute résistance à la corrosion, qualités mécaniques pour les céramiques denses, et propriétés électriques, ioniques pour leur usage industriel. Il existe différentes familles de céramiques en fonction de leur composition, dont les phosphates de calcium qui sont des céramiques bioactives.

L'implantation de l'implant injectable dans l'organisme a essentiellement pour but de faire apparaître un tissu de nouvelles fibres de collagène, apparition généralement qualifiée de néo-collagénèse, responsable du comblement de la ridule ou de la dépression cutanée. La démarche suivie est de faire démarrer le mécanisme, c'est-à-dire la synthèse de nouvelles fibres de collagène, mais sans pour autant que l'implant subsiste trop longtemps dans l'organisme. En effet, tout corps étranger implanté induit une réaction inflammatoire à corps étranger non spécifique qui dans cette indication est recherchée à moyen terme. Le choix d'un composé de céramique présentant une durée de résorbabilité telle que définie ci-dessus permet avantageusement d'allier le maximum d'efficacité avec un minimum de risques.

En effet, aucun implant non résorbable ne paraît généralement souhaitable. Ainsi, avantageusement selon l'invention, le composé de céramique qui constitue une phase minérale se dégrade ou se solubilise pratiquement totalement après injection sous-cutanée ou intradermique puis est pratiquement totalement éliminé de l'organisme par les processus naturels.

En outre, l'implant selon l'invention allie avantageusement la commodité d'emploi, la seringabilité du produit, la résorbabilité en un temps contrôlé du vecteur comme du composé de céramique, l'absence d'allergénicité du produit (par absence de composé d'origine animale), qui rend tout test préalable inutile.

Selon l'invention, le composé de céramique comporte généralement une surface spécifique de 0,5 m²/g à 100 m²/g, de préférence de 2 m²/g à 27 m²/g. La surface spécifique est généralement mesurée par la méthode BET.

L'invention concerne aussi un implant injectable tel que les microparticules sont présentes dans le fluide vecteur dans une proportion poids/ volume strictement supérieure à 0% et inférieure à 15%, de préférence de 2 à 12%.

Selon l'invention, le composé de céramique comprend généralement au moins un élément choisi dans le groupe formé par le phosphate tricalcique (βTCP) et les produits biphasés (BPC) qui comprennent de l'hydroxyapatite (HAP) et du βTCP en proportion variable, de préférence ledit élément étant du βTCP, à condition que lesdites microparticules soient biodégradables, de préférence biorésorbables. Ainsi, des microparticules de HAP sont exclues selon l'invention.

L'hydroxyapatite (HAP), de formule générale Ca₁₀(PO₄)₆(OH)₂ est le plus proche des cristaux d'apatite biologique. Le rapport atomique Ca/P (1,67) est généralement inférieur à celui de l'os. Le phosphate tricalcique (β PTC) est de formule Ca₃(PO₄)₂. Le rapport Ca/P est généralement de 1,5. Les produits biphasés (BPC) associent dans un rapport variable de l'HAP et du βPTC. Il est à noter que la préparation de ces produits fait généralement intervenir de nombreuses variables qui conditionnent leur comportement biologique : composition élémentaire, nature des phases minérales, micro et macroporosité, présence d'impuretés.

Dans ce mode de réalisation particulièrement préféré selon l'invention, le composé de céramique selon l'invention est bioactif, et a donc des échanges chimiques avec les tissus vivants. Par bioactivité, on entend selon l'invention une propriété permettant des réactions chimiques spécifiques, à l'interface implant-tissu récepteur. Elle dépend directement des propriétés chimiques et physico-chimiques du matériau, et s'oppose à la bioinertie (propriété de matériaux biocompatibles mais inertes). Après implantation par injection, le composé est généralement le siège d'une dissolution extracellulaire et d'une dégradation d'origine cellulaire, dépendant de la structure chimique (βTCP, BCP) physique (pores du matériau), et de l'environnement du matériau. Les fluides biologiques, dont le fluide vecteur, occupant les micropores du composé de céramique s'enrichissent en calcium. La dégradation qui est de préférence une résorption de l'implant selon l'invention ne doit généralement pas être trop rapide pour permettre une réaction inflammatoires à corps étranger non spécifique responsable de la synthèse de nouvelle fibres de collagène. L'HAP est très peu soluble et son taux de dégradation est généralement très bas in vivo, mais varie en fonction du pH. Le βTCP est beaucoup plus soluble et présente généralement une dégradation in vivo importante. Les produits biphasés ont des propriétés qui varient en fonction de rapport entre HAP et βTCP. Le résultat de l'implantation dépend donc le plus souvent de la cinétique de colonisation et de résorption, qui est généralement conditionnée par les caractères chimiques et physico-chimiques de l'implant injectable selon l'invention ; avantageusement, ces critères sont contrôlés grâce à la nature de l'implant selon l'invention.

Par exemple les microparticules de céramique sont des particules de BIOSORB® de la société, vendus comme particules de βTCP par la société SBM.

Le fluide vecteur présente généralement une résorbabilité limitée, typiquement d'environ un à environ quatre mois.

De préférence, le fluide vecteur de l'implant est un gel biocompatible, de préférence biorésorbable.

Dans un mode de réalisation préféré de l'invention, le fluide vecteur est tel que le composé à base d'acide hyaluronique comprend en majeure partie de l'acide hyaluronique. Par à base de, on entend selon l'invention qu'au moins la majeure partie dudit composé est de l'acide hyaluronique, réticulé ou non, ou un de ses sels ou un de ses dérivés polysaccharidiques.

Dans un mode de réalisation préféré de l'invention, ledit composé à base d'acide hyaluronique comprend de l'acide hyaluronique de masse moléculaire supérieure à un million de daltons, de préférence de un million à cinq millions de daltons.

Le fluide vecteur peut aussi comprendre en outre au moins un élément choisi dans le groupe formé par les dérivés cellulosiques tels que CMC (Carboxy Méthyl Cellulose), HPMC (Hydroxy Propyl Méthyl Cellulose), HPC (Hydroxy Propyl Cellulose) et les autres glycosaminoglycanes que l'acide hyaluronique.

L'implant selon l'invention se présente sous la forme de microparticules, éventuellement sous forme de microsphères, en suspension dans un fluide vecteur véhiculant lesdites microparticules. Ces microparticules doivent avoir un diamètre supérieur à 10 µm, afin d'éviter une phagocytose rapide ou immédiate par les macrophages. Elles doivent avoir un diamètre inférieur à 45 µm, de façon à pouvoir être injectées par une aiguille très fine (typiquement de gauge 25 à gauge 30). Avantageusement et selon l'invention, le fluide vecteur est choisi pour présenter une viscosité intrinsèque suffisante pour être injecté à travers une aiguille de gauge 25 à 30, par exemple de 1500 à 4000 m³/kg à 25°C, et pour maintenir de façon homogène la phase minérale qu'est le composé de céramique en suspension dans la phase fluide qu'est le fluide vecteur.

Le composé de céramique selon l'invention peut être préparé selon tout procédé connu de l'homme du métier. Deux types de procédés peuvent être distingués, selon que le composé de céramique est d'origine synthétique ou naturelle (biologique). La préparation du premier type de procédé, pour le composé de céramique d'origine synthétique, est telle que décrite ci-après. Les produits de base sont préparés par synthèse chimique et se présentent sous forme de poudre. La mise en forme pour l'utilisation en injection sous-cutanée ou intradermique (porosité et forme) nécessite différentes opérations après calcination, à température généralement inférieure à 900°C. Ainsi, la poudre peut être compactée sous pression puis chauffée, à température généralement de 1100 à 1500°C, ce qui produit au moins partiellement un frittage des constituants de la poudre. Il y a alors fusion desdits constituants, puis agglomération de microcristaux qui se forment au refroidissement et restent soudés. Les interstices entre ces microcristaux déterminent une microporosité, c'est-à-dire une taille de microparticules au moins partiellement de taille généralement inférieure à 5 µm. La microporosité dépend à la fois de la pression et de la température. L'addition à la poudre par exemple de billes de naphtalène peut avantageusement créer une macroporosité, c'est-à-dire une taille de microparticules au moins partiellement supérieure généralement à 100 µm. Le diamètre des macropores est déterminé par le diamètre desdites billes, qui se subliment à haute température.

En ce qui concerne la préparation du second type de procédé, pour le composé de céramique d'origine naturelle, elle est généralement telle que explicité ci-dessus pour le premier type de préparation, c'est-à-dire selon un traitement thermique identique, mais à partir de structures biologiques le plus souvent phospho-calciques poreuses pré-existantes (corail, os). Ledit traitement thermique détruit les éléments organiques et provoque une céramisation de la trame phospho-calcique.

La caractérisation physico-chimique du composé de céramique selon l'invention est généralement telle que connue de l'homme du métier. Elle peut être effectuée par analyse élémentaire, par exemple par dosage (du calcium (Ca), du phosphore (P), et du ou des éléments traces,), par recherche du ou des éléments lourds éventuellement présents (pollution) et/ou par détermination du rapport Ca/P. Elle peut être aussi effectuée, en complément ou non de l'analyse élémentaire, par diffraction aux rayons X par exemple par détermination des phases minérales (HAP, βTCP),

Par détermination de la cristallinité (taille, forme des cristaux), et/ou par recherche de défauts cristallins. Elle peut être aussi effectuée, en complément ou non de l'analyse élémentaire et/ou de la diffraction aux rayons X, par spectrométrie infra-rouge, par exemple par détermination de groupes fonctionnels (carbonates, présence d'eau, composants organiques, substitutions ioniques...), par détermination de structure (détermination des états de surfaces, de la micro et macroporosité).

L'invention concerne aussi un procédé de préparation d'un implant injectable selon l'invention, comprenant les étapes suivantes :
- on prépare dans une étape préalable un composé de céramique biocompatible sous forme de microparticules telles que définies précédemment,
- on prépare dans une autre étape, indépendamment de l'étape préalable précédente, une solution d'un fluide vecteur comportant au moins un composé à base d'acide hyaluronique et au moins un composé thixotrope biodégradable ayant des propriétés pseudoplastiques,
- on introduit ensuite le composé de céramique de l'étape préalable dans le fluide vecteur de l'autre étape, dans une étape finale, de manière à obtenir une suspension essentiellement homogène, typiquement par utilisation d'un moyen d'homogénéisation de type mélangeur.

Par solution d'un fluide vecteur on entend selon l'invention un mélange d'un fluide vecteur éventuellement dans un solvant, de préférence aqueux.

L'implant injectable selon l'invention peut se présenter sous la forme d'une seringue pré-remplie prête à l'emploi, d'un flacon pré-rempli prêt à l'emploi, ou d'un lyophilisat à reconstituer extemporanément.

L'invention concerne aussi un kit de mise en oeuvre extemporanée d'un implant selon l'invention tel qu'il comprend au moins un composé de céramique biocompatible et au moins un fluide vecteur.

Le kit selon l'invention comprend généralement dans une première partie le composé de céramique et dans une seconde partie le vecteur, et permet ainsi lors de son utilisation la reconstitution de l'implant injectable selon l'invention.

L'invention concerne l'utilisation d'un implant injectable selon l'invention pour le comblement des rides et/ou ridules et/ou dépressions cutanées et/ou cicatrices, comprenant l'injection sous-cutanée d'un tel implant. Cela s'applique aussi bien au corps humain qu'au corps animal. Une telle utilisation se situe donc principalement dans le domaine de la chirurgie réparatrice ou plastique, ou dans le domaine de la dermatologie esthétique.

### Exemples

Les différents composés exemplifiés entrant dans la formulation de nos produits sont les suivants :
βTCP
Hyaluronate de sodium
Xanthane
Ils ont été choisis pour leur caractère résorbables, leurs propriétés viscosifiantes et stabilisantes dans les suspensions.

### Exemples de formulations :

| | | | |
|---|---|---|---|
| βTCP | 10% (P/V) | βTCP | 7% (P/V) |
| Hyaluronate de sodium | 2% | Hyaluronate de sodium | 2,2% |
| Xanthane | 0,5% | Xanthane | 0,5% |
| | | | |
| βTCP | 10% (P/V) | βTCP | 7% (P/V) |
| Hyaluronate de sodium | 2,2% | Hyaluronate de sodium | 2,2% |
| Xanthane | 0,6% | Xanthane | 0,6% |
| | | | |
| βTCP | 10% (P/V) | βTCP | 7% (P/V) |
| Hyaluronate de sodium | 1,8% | Hyaluronate de sodium | 1,8% |
| Xanthane | 1% | Xanthane | 1% |
| | | | |
| βTCP | 10% (P/V) | βTCP | 7% (P/V) |
| Hyaluronate de sodium | 1,6% | Hyaluronate de sodium | 1,6% |
| Xanthane | 0,8% | Xanthane | 0,8% |

Les microparticules de céramique qui ont été utilisée dans ces exemples sont des particules de BIOSORB® de la société, vendus comme particules de βTCP par la société SBM.

Elles sont parfaitement tolérées et donc biocomptatibles.

Les études de cytotoxicité, de sensibilisation, d'irritation et d'implantation chez l'animal selon la norme ISO 10993 démontrent une excellente tolérance des formulations exemplifiées selon l'invention. Une étude de toxicité aiguë en injection intrapéritonéale a démontré que la dose létale chez la souris est supérieure à 10ml/Kg, le produit testé (βTCP en suspension dans un gel d'acide hyaluronique et de xantane) ne présente pas de toxicité et satisfait au test conformément à la norme ISO 10993.
Par opposition aux céramiques bio-inertes (alumine, zircone), le βTCP est une céramique bioactive, et a donc des échanges chimiques avec les tissus vivants.

Par opposition à l'hydroxyapatite (HAP), le βTCP est beaucoup plus soluble et présente une dégradation in vivo importante.

La résorption de l'implant selon l'invention ne doit pas être trop rapide pour permettre une colonisation des microparticules par les macrophages. Alors démarre une réaction inflammatoire à corps étranger non spécifique qui aboutit à une encapsulation par du tissu fibreux.

Le résultat clinique dépend donc de la cinétique de colonisation et de résorption, qui est conditionnée par les caractères chimiques et physico-chimiques de l'implant; ces critères doivent généralement être parfaitement contrôlés.

Le βTCP n'a pas encore été évalué dans le comblement des rides (injection intradermique). Cependant, de nombreuses applications dans les tissus mous (parodontie, régénération tissulaire guidée) et dans les tissus osseux ont mis en évidence la bonne tolérance du βTCP, tant chez l'animal que chez l'homme. Des formulations d'application topique ont été testées chez le rat. Celle-ci n'a pas mis en évidence de phénomène d'irritation, de sensibilisation.

Après implantation, le matériau est le siège d'une dissolution extracellulaire et d'une dégradation d'origine cellulaire.

Une étude d'implantation chez le rat a été menée pendant 3 mois pour évaluer les effets des formulations selon l'invention en injection intradermique. Celles-ci ont montré la parfaite innocuité du produit au cours et au décours de l'injection (aucune douleur, aucune irritation). Une étude histologique a démontré après implantation sous cutanée après un recul de 3 mois, aucune lésion macroscopique quelque soit les concentrations en βTCP utilisées.

Des réactions inflammatoires à corps étranger non spécifiques sont observées en accord avec les données de la littérature. De plus celles-ci, localisées uniquement au contact du matériau, démontrent la parfaite tolérance du produit testé. Aucune abcédation ou nécrose tissulaire n'a été observée autour de ces implants.
• A un mois, on observe une capsule tissulaire conjonctive, vascularisée entourant l'implant. Cette capsule est constituée principalement de cellules conjonctives, macrophages, lymphocytes, cellules géantes à corps étranger et de mastocytes.
• A 3 mois, on note une nette diminution de la densité cellulaire de la capsule conjonctive, ainsi que son épaisseur traduisant une diminution de l'intensité de la réaction inflammatoire au fur et à mesure que l'implant se dégrade (environ 50% a 3 mois ce qui prouve que le produit aura totalement disparu entre 8 et 16 mois selon les modèles de dégradation connus). Seul le nombre de mastocytes reste inchangé. Par contre, on remarque une augmentation du dépôt de fibres de collagène.

### Rationnel de développement

Les différentes formules ont été évaluée avec et sans xanthane puis ont été soumises à des cycles d'autoclavage (121°C 20') afin d'évaluer l'influence du xanthane sur la stabilisation de suspension. Une partie de ces préparations ont été formulées à partir d'eau pour préparations injectables, d'autres à partir de solution de chlorure de sodium à 0,9% afin de mesurer l'incidence d'une solution saline sur la viscosité des gels préparés après un cycle de stérilisation par la vapeur. Ces études ont permis de révéler le très bon pouvoir de suspension du xanthane ainsi qu'une meilleure résistance à la chaleur en présence d'une solution saline concentrée à 0,9%. Les documents de données de stabilité actuelle ou pré stabilité confirment une stabilité des suspensions autoclavées préparées avec 0,5% et 0,6% de xanthane dans un gel d'acide hyaluronique à 2%.

Le xanthane s'oppose à la sédimentation des dispersions en raison de son seuil d'écoulement (ou contrainte de cisaillement) très élevé. Cette caractéristique, et la viscosité induite sont bien supérieures à celles des gommes végétales (guar, caroube), des celluloses greffées, ou des alginates.

La propriété fondamentale du xanthane est son action de contrôle de la rhéologie des systèmes aqueux et son effet stabilisant des systèmes multiphasiques aqueux, qu'il s'agisse de stabiliser un liquide (émulsions), un solide (suspensions), ou un gaz (mousses).

Le comportement rhéologique du xanthane a par ailleurs comme caractéristique une pseudo plasticité élevée, soit un comportement visqueux réversible au cisaillement, supérieure à celle des polysaccharides comme l'acide hyaluronique cité ci-dessus.

Il présente une résistance à l'hydrolyse enzymatique y compris des galactomannanases, amylases, cellulases, pectinases, protéases...

Toutes ces caractéristiques font du Xanthane un adjuvant de choix pour les formulations injectables à visée esthétique et dermatologique.

## Revendications

1. Implant injectable par voie sous-cutanée ou intradermique dans le tissu fibreux, comprenant des microparticules d'au moins un composé de céramique biocompatible en suspension dans au moins un fluide vecteur, ledit implant étant **caractérisé en ce que** lesdites microparticules sont biodégradables et ont une taille de 10 à 80 µm, de préférence de 15 à 50 µm, ledit composé de céramique comprenant au moins un élément choisi dans le groupe formé par le phosphate tricalcique (βTCP) et les produits biphasés (BPC) qui comprennent de l'HAP et du βTCP en proportion variable, de préférence ledit élément étant du βTCP, et **en ce que** ledit fluide vecteur comprend au moins un composé à base d'acide hyaluronique et au moins un composé thixotrope biodégradable ayant des propriétés pseudoplastiques.

2. Implant selon la revendication 1, **caractérisé en ce que** ledit composé de céramique comporte une surface spécifique de 0,5 m²/g à 100 m²/g, de préférence de 2 m²/g à 27 m²/g.

3. Implant injectable selon la revendication 1 ou 2, **caractérisé en ce que** les microparticules sont biorésorbables, une fois l'implantation effectuée dans le tissu fibreux, dans un délai de 2 à 36 mois, de préférence de 3 à 24 mois.

4. Implant injectable selon la revendication 3, **caractérisé en ce que** les microparticules sont biorésorbables, une fois l'implantation effectuée dans le tissu fibreux, dans un délai de 4 à 18 mois.

5. Implant injectable selon l'une des revendications 1 à 4, **caractérisé en ce que** les microparticules sont présentes dans le fluide vecteur dans une proportion poids/volume strictement supérieure à 0% et inférieure à 15%, de préférence de 2 à 12%.

6. Implant injectable selon l'une des revendications 1 à 5, **caractérisé en ce que** le fluide vecteur de l'implant est un gel biocompatible de préférence biorésorbable.

7. Implant injectable selon l'une des revendications 1 à 6, **caractérisé en ce que** le composé à base d'acide hyaluronique comprend en majeure partie de l'acide hyaluronique.

8. Implant injectable selon la revendication précédente, **caractérisé en ce que** ledit composé à base d'acide hyaluronique comprend de l'acide hyaluronique de masse moléculaire supérieure à un million de daltons, de préférence de un million à cinq millions de daltons.

9. Implant injectable selon l'une des revendications 1 à 8, **caractérisé en ce que** ledit fluide vecteur comprend comme composé thixotrope biodégradable ayant des propriétés pseudoplastiques au moins un élément choisi dans le groupe formé par les dérivés cellulosiques.

10. Implant injectable selon la revendication 9, **caractérisé en ce que** les dérivés cellulosiques sont choisis parmi : CMC (Carboxy Méthyl Cellulose), HPMC (Hydroxy Propyl Méthyl Cellulose) et HPC (Hydroxy Propyl Cellulose).

11. Implant selon la revendication 10, **caractérisé en ce que** le dérivé cellulosique est une carboxyméthylcellulose.

12. Implant injectable selon l'une des revendications 1 à 11 se présentant sous la forme d'une seringue pré-remplie prête à l'emploi, d'un flacon pré-rempli prêt à l'emploi, ou d'un lyophilisat à reconstituer.

13. Procédé de préparation d'un implant injectable selon l'une des revendications 1 à 11, comprenant les étapes suivantes :
- on prépare dans une étape préalable un composé de céramique biocompatible sous forme de microparticules défini selon la revendication 1,
- on prépare dans une autre étape, indépendamment de l'étape préalable précédente, une solution d'un fluide vecteur comportant au moins un composé à base d'acide hyaluronique et au moins un composé thixotrope biodégradable ayant des propriétés pseudoplastiques,
- on introduit ensuite le composé de céramique de l'étape préalable dans le fluide vecteur de l'autre étape, dans une étape finale, de manière à obtenir une suspension essentiellement homogène.

14. Kit de mise en oeuvre extemporanée d'un implant injectable selon l'une des revendications 1 à 12 comprenant dans une première partie le composé de céramique et dans une seconde partie le fluide vecteur.

15. Implant injectable selon l'une des revendications 1 à 12 pour le comblement de rides et/ou ridules et/ou dépressions cutanées et/ou cicatrices, comprenant l'injection sous-cutanée d'un tel implant.

## Claims

1. Implant that can be injected subcutaneously or intradermally into fibrous tissue, comprising microparticles of at least one biocompatible ceramic compound in suspension in at least one liquid vector, the said implant being **characterized in that** the said microparticles are biodegradable and have a size of 10 to 80 µm, preferably from 15 to 50 µm, the said ceramic compound comprising at least one element chosen from among the group formed by tricalcium phosphate (βTCP) and the biphasic products (BPC) that comprise PAH and βTCP in a variable proportion, the said element preferably being βTCP, and **in that** the said liquid vector comprises at least one compound based on hyaluronic acid, and at least one biodegradable thixotropic compound having pseudoplastic properties.

2. Implant according to claim 1, **characterized in that** the said ceramic compound has a specific surface of 0.5 m²/g to 100 m²/g, preferably 2 m²/g to 27 m²/g.

3. Injectable implant according to claim 1 or 2, **characterized in that** the microparticles are bioresorbable, once implantation into fibrous tissue has taken place, over a time period of 2 to 36 months, preferably 3 to 24 months.

4. Injectable implant according to claim 3, **characterized in that** the microparticles are bioresorbable, once implantation into fibrous tissue has taken place, over a time period of 4 to 18 months.

5. Injectable implant according to any of claims 1 to 4, **characterized in that** the microparticles are present in the liquid vector in a weight/volume proportion of strictly greater than 0% and less than 15%, preferably 2 to 12%.

6. Injectable implant according to any of claims 1 to 5, **characterized in that** the liquid vector of the implant is a biocompatible gel, preferably a bioresorbable one.

7. Injectable implant according to any of claims 1 to 6, **characterized in that** the compound based on hyaluronic acid comprises primarily hyaluronic acid.

8. Injectables implant according to the preceding claim, **characterized in that** the said compound based on hyaluronic acid comprises hyaluronic acid having a molecular mass of greater than one million daltons, preferably one million to five million daltons.

9. Injectable implant according to any of claims 1 to 8, **characterized in that** the said liquid vector comprises at least one element chosen from among the group formed by cellulose derivatives as the biodegradable thixotropic compound having pseudoplastic properties.

10. Injectable implant according to claim 9, **characterized in that** the cellulose derivatives are chosen from among:
CMC (carboxymethyl cellulose), HPMC (hydroxypropylmethyl cellulose), and HPC (hydroxypropyl cellulose).

11. Implant according to claim 10, **characterized in that** the cellulose derivative is a carboxymethyl cellulose.

12. Injectable implant according to any of claims 1 to 11, presenting itself in the form of a pre-filled ready-to-use syringe, a pre-filled ready-to-use vial, or a lyophilisate that must be reconstituted.

13. Process for the preparation of an injectable implant according to any of claims 1 to 11, comprising the following stages:
- in a preliminary stage, a biocompatible ceramic compound is prepared in the form of microparticles as defined according to claim 1,
- in another stage, independent of the preceding preliminary stage, a solution of a liquid vector comprising at least one compound based on hyaluronic acid and at least one biodegradable thixotropic compound having pseudoplastic properties is prepared,
- subsequently, the ceramic compound from the preliminary stage is introduced into the liquid vector of the other stage, in a final stage, in such a manner as to obtain an essentially homogeneous suspension.

14. Kit for extemporaneous use of an injectable implant according to any of claims 1 to 12, comprising, in a first part, the ceramic compound, and, in a second part, the liquid vector.

15. Injectable implant according to any of claims 1 to 12, for filling out wrinkles and fine lines and/or pits and/or scars, wherein the implant is subcutaneously injected.

## Patentansprüche

1. Subkutan oder intrakutan in das Fasergewebe injizierbare Implantat, welches Mikropartikel aus mindestens einer biokompatiblen Keramikverbindung, die in mindestens einem flüssigen Vektor suspendiert ist, umfasst, wobei das besagte Implantat **dadurch gekennzeichnet ist, dass** die besagten Mikropartikel biologisch abbaubar sind und eine Größe von 10 bis 80 µm, vorzugsweise 15 bis 50 µm besitzen, wobei die besagte Keramikverbindung mindestens ein aus der aus Trikalziumphosphat (βTCP) und den biphasigen Produkten (BPC), welche PAH und βTCP in variabler Proportion enthalten, gebildeten Gruppe ausgewähltes Element umfasst, wobei das besagte Element vorzugsweise βTCP ist, und **dadurch**, dass der besagte flüssige Vektor mindestens eine Verbindung auf der Basis von Hyaluronsäure umfasst, sowie mindestens eine biologisch abbaubare thixotrope Verbindung, die pseudoplastische Eigenschaften besitzt.

2. Implantat gemäß Patentanspruch 1, **dadurch gekennzeichnet, dass** die besagte Keramikverbindung eine spezifische Oberfläche von 0.5 m²/g bis 100 m²/g, vorzugsweise 2 m²/g bis 27 m²/g aufweist.

3. Injizierbares Implantat gemäß Patentanspruch 1 oder 2, **dadurch gekennzeichnet, dass** die Mikropartikel bioresorbierbar sind, nachdem die Implantation in das Fasergewebe stattgefunden hat, über einen Zeitraum von 2 bis 36 Monaten, vorzugsweise 3 bis 24 Monaten.

4. Injizierbares Implantat gemäß Patentanspruch 3, **dadurch gekennzeichnet, dass** die Mikropartikel bioresorbierbar sind, nachdem die Implantation in das Fasergewebe stattgefunden hat, über einen Zeitraum von 4 bis 18 Monaten.

5. Injizierbares Implantat gemäß einem der Patentansprüche 1 bis 4, **dadurch gekennzeichnet, dass** die Mikropartikel in dem flüssigen Vektor in einer Proportion von Gewicht/Volumen vorhanden sind, die auf jeden Fall mehr als 0% und weniger als 15% beträgt, vorzugsweise 2 bis 12%.

6. Injizierbares Implantat gemäß einem der Patentansprüche 1 bis 5, **dadurch gekennzeichnet, dass** der flüssige Vektor des Implantats ein biokompatibles, vorzugsweise ein resorbierbares Gel ist.

7. Injizierbares Implantat gemäß einem der Patentansprüche 1 bis 6, **dadurch gekennzeichnet, dass** die Verbindung auf der Basis von Hyaluronsäure vorwiegend Hyaluronsäure beinhaltet.

8. Injizierbares Implantat gemäß dem vorhergehenden Patentanspruch, **dadurch gekennzeichnet, dass** die besagte verbindung auf der Basis von Hyaluronsäure die Hyaluronsäure mit einem Molekulargewicht von mehr als einer Million Dalton, vorzugweise einer Million bis fünf Millionen Dalton enthält.

9. Injizierbares Implantat gemäß einem der Patentansprüche 1 bis 8, **dadurch gekennzeichnet, dass** der besagte flüssige Vektor als biologisch abbaubare Verbindung mit pseudoplastischen Eigenschaften mindestens ein aus der aus Zellulosederivaten gebildeten Gruppe ausgewähltes Element umfasst.

10. Injizierbares Implantat gemäß Patentanspruch 9, **dadurch gekennzeichnet, dass** die Zellulosederivate ausgewählt sind unter:
CMC (Karboxymethylzellulose), HPMC (Hydroxypropylmethylzellulose) und HPC (Hydroxypropylzellulose).

11. Implantat gemäß Patentanspruch 10, **dadurch gekennzeichnet, dass** das Zellulosederivat eine Karboxymethylzellulose ist.

12. Injizierbares Implantat gemäß einem der Patentansprüche 1 bis 11, das in Form einer vorgefüllten gebrauchsfertigen Spritze, eines vorgefüllten gebrauchsfertigen Fläschchens, oder eines wiederauffüllbaren Lyophilisats vorliegt.

13. Verfahren für die Zubereitung eines injizierbaren Implantats gemäß einem der Patentansprüche 1 bis 11, welches die folgenden Schritte umfasst:
- in einem vorausgehenden Schritt wird eine biokompatible Keramikverbindung in Form von Mikropartikeln zubereitet, wie sie gemäß Patentanspruch 1 definiert sind,
- in einem weiteren Schritt, unabhängig von dem vorhergehenden vorausgehenden Schritt, wird eine Lösung eines flüssige Vektoren, welcher mindestens eine Verbindung auf der Basis von Hyaluronsäure und mindestens eine biologisch abbaubare thixotrope Verbindung mit pseudoplastischen Eigenschaften umfasst, zubereitet,
- daraufhin wird die Keramikverbindung aus dem vorausgehenden Schritt in einem abschließenden Schritt mit dem flüssigen Vektor aus dem anderen Schritt zusammengeführt, auf solche Weise, dass eine im Wesentlichen homogene Suspension erhalten wird.

14. Kit zur unvorbereiteten Verwendung eines injizierbaren Implantats gemäß einem der Patentansprüche 1 bis 12, welches die Keramikverbindung in einem ersten Teil und den flüssigen Vektoren in einem zweiten Teil enthält.

15. Injizierbares Implantat gemäß einem der Patentansprüche 1 bis 12 zum Auffüllen von Falten und Fältchen und/oder Hautvertiefungen und/oder Narben, das die subkutane Injektion eines solches Implantats umfasst.
